# EUROPEAN PATENT APPLICATION

(11) **EP 1 994 932 A1**
(43) Date of publication of application: **26.11.2008**
(21) Application number: 08016004.7
(22) Date of filing: 21.01.2004
(51) Int. Cl.: A61K 31/5513, A61P 25/18

(54) **Use of N-desmethylclozapine to treat human psychosis**

(30) Priority: 23.01.2003 US 442690 P
(62) Divisional of application: 04704073.8
(71) Applicant: Acadia Pharmaceuticals Inc., San Diego CA 92121-1402 (US)
(72) Inventor: The designation of the inventor has not yet been filed
(74) Representative: Plougmann & Vingtoft A/S

(57) **Abstract**

Disclosed herein is a method to treat psychosis. Treatment is carried out by administering a therapeutically effective amount of N-desmethylclozapine to a patient suffering from psychosis.

## Description

### Related Application

The present invention relates to the discovery of potent muscarinic receptor agonist properties of the dibenzodiazepine compound N-desmethylclozapine, 8-chloro-11-(1-piperazinyl)-5H-dibenzo[b,e][1,4]diazepine, which supports the clinical use of this drug as a superior therapeutic agent for the treatment of pain, glaucoma, dementia, affective disease, and psychosis.

### Background of the Invention

The physiological actions of the hormone/neurotransmitter acetylcholine are mediated, in part, by muscarinic acetylcholine receptors. Muscarinic receptors comprise a family of five (M1-M5) transmembrane proteins that mediate slow, modulatory signalling in cells and tissues expressing these genes. Muscarinic receptors are the targets of a number of therapeutically useful agents (1, 2). Peripherally, muscarinic receptors mediate the actions of acetylcholine in the parasympathetic nervous system. Peripherally acting muscarinic receptor agonists are therapuetically useful in lowering intra-ocular pressure in patients with glaucoma (3). Compounds that potentiate the central actions of acetylcholine as well as centrally acting muscarinic receptor agonists have both demonstrated clinical utility in the treatment of a number of neuropsychiatric diseases (1, 2, 4-7).

The actions of acetylcholine are terminated by degradation of the molecule by acetylcholinesterase enzymes. Inhibition of these enzymes within the central nervous system leads to increased concentrations of acetylcholine at muscarinic receptors. A number of acetylcholinesterase inhibitors have been developed and are in routine clinical use as cognitive enhancing agents in dementia (4).

A number of centrally acting muscarinic agonist have been the subject of clinical testing. One of these, Xanomeline, has been shown to possess efficacy in controlling psychosis and related behavioral disturbances observed in Alzheimer's Disease patients (5). Further, it has recently been demonstrated that xanomeline is efficacious in treating schizophrenia (6). Interestingly, it displayed efficacy against both positive and negative symptoms, and did not induce adverse motoric effects in initial clinical studies in schizophrenics. These data suggest that compounds with muscarinic receptor agonist properties are likely to be efficacious in treating the behavioral disturbances common to neurodegenerative disease such as Alzheimers Disease and as antipsychotics to treat human psychoses, but only if they are tolerated in these patient populations. Additionally, muscarinic receptor agonists have shown activity in pre-clinical models of neuropathic pain states (7).

### Summary of the Invention

Disclosed herein is the use of N-desmethylclozapine in the preparation of a medicament for ameliorating psychosis. In some embodiments, the medicament is suitable for administration to a human. In some embodiments, the medicament is administered as a single dose. In other embodiments, the medicament is administered as a plurality of doses. In some embodiments, the medicament also comprises an additional therapeutic agent. In some embodiments, the additional therapeutic agent is selected from the group consisting of selective serotonin reuptake inhibitors, norepinephrine reuptake inhibitors, dopamine agonists, antipsychotic agents, and inverse serotonin 2A agonists.

Also disclosed herein is use of N-desmethylclozapine in the preparation of a medicament for ameliorating affective disorders. In some embodiments, the medicament is suitable for administration to a human. In one embodiment, the affective disorder is depression. In another embodiment, the affective disorder is mania. In some embodiments, the medicament is administered as a single dose. In other embodiments, the medicament is administered as a plurality of doses. In some embodiments, the medicament also comprises an additional therapeutic agent. In some embodiments, the additional therapeutic agent is selected from the group consisting of selective serotonin reuptake inhibitors, norepinephrine reuptake inhibitors, dopamine agonists, antipsychotic agents, and inverse serotonin 2A agonists.

Also disclosed herein is use of N-desmethylclozapine in the preparation of a medicament for ameliorating dementia. In some embodiments, the medicament is suitable for administration to a human. In some embodiments, the medicament is administered as a single dose. In other embodiments, the medicament is administered as a plurality of doses. In some embodiments, the dementia manifests as a cognitive impairment. In some embodiments, the dementia manifests as a behavioral disturbance. In some embodiments, the medicament also comprises an additional therapeutic agent. In some embodiments, the additional therapeutic agent is selected from the group consisting of selective serotonin reuptake inhibitors, norepinephrine reuptake inhibitors, dopamine agonists, antipsychotic agents, and inverse serotonin 2A agonists.

Also disclosed herein is use of N-desmethylclozapine in the preparation of a medicament for ameliorating neuropathic pain. In some embodiments, the medicament is suitable for administration to a human. In some embodiments, the medicament is administered as a single dose. In other embodiments, the medicament is administered as a plurality of doses. In some embodiments, the medicament also comprises an additional therapeutic agent. In some embodiments, the additional therapeutic agent is selected from the group consisting of selective serotonin reuptake inhibitors, norepinephrine reuptake inhibitors, dopamine agonists, antipsychotic agents, and inverse serotonin 2A agonists.

Also disclosed herein is use of N-desmethylclozapine in the preparation of a medicament for ameliorating the symptoms of glaucoma. In some embodiments, the medicament is suitable for administration to a human. In some embodiments, the medicament is administered as a single dose. In other embodiments, the medicament is administered as a plurality of doses. In various embodiments, the symptoms of glaucoma are selected from the group consisting of elevated intraocular pressure, optic nerve damage, and decreased field of vision. In some embodiments, the medicament also comprises an additional therapeutic agent. In some embodiments, the additional therapeutic agent is selected from the group consisting of selective serotonin reuptake inhibitors, norepinephrine reuptake inhibitors, dopamine agonists, antipsychotic agents, and inverse serotonin 2A agonists.

Also disclosed herein is a method of treating psychosis comprising: identifying a subject suffering from one or more symptoms of psychosis; and contacting the subject with a therapeutically effective amount of N-desmethylclozapine; whereby the one or more symptoms of psychosis are ameliorated. In one embodiment, the subject is human. In some embodiments, the therapeutically effective amount of N-desmethylclozapine is administered as a single dose. In other embodiments, the therapeutically effective amount of N-desmethylclozapine is administered as a plurality of doses. In one embodiment, the method further comprises contacting the subject with an additional therapeutic agent. In one embodiment, the subject is contacted with the additional therapeutic agent subsequent to the contacting with N-desmethylclozapine. In another embodiment, the subject is contacted with the additional therapeutic agent prior to the contacting with N-desmethylclozapine. In still another embodiment, the subject is contacted with the additional therapeutic agent substantially simultaneously with N-desmethylclozapine. In some embodiments, the additional therapeutic agent is selected from the group consisting of selective serotonin reuptake inhibitors, norepinephrine reuptake inhibitors, dopamine agonists, antipsychotic agents, and inverse serotonin 2A agonists.

Also disclosed herein is a method of treating affective disorders comprising: identifying a subject suffering from one or more symptoms of an affective disorder; and administering a therapeutically effective amount of N-desmethylclozapine to the subject, whereby the one or more symptoms of the affective disorder are ameliorated. In one embodiment, the subject is human. In one embodiment, the affective disorder is depression. In another embodiment, the affective disorder is mania. In some embodiments, the therapeutically effective amount of N-desmethylclozapine is administered as a single dose. In other embodiments, the therapeutically effective amount of N-desmethylclozapine is administered as a plurality of doses. In one embodiment, the method further comprises administering to the subject an additional therapeutic agent. In one embodiment, the subject is contacted with the additional therapeutic agent subsequent to the contacting with N-desmethylclozapine. In another embodiment, the subject is contacted with the additional therapeutic agent prior to the contacting with N-desmethylclozapine. In still another embodiment, the subject is contacted with the additional therapeutic agent substantially simultaneously with N-desmethylclozapine. In some embodiments, the additional therapeutic agent is selected from the group consisting of selective serotonin reuptake inhibitors, norepinephrine reuptake inhibitors, dopamine agonists, antipsychotic agents, and inverse serotonin 2A agonists.

Also disclosed herein is a method of treating dementia, comprising: identifying a subject suffering from one or more symptoms of dementia; and administering a therapeutically effective amount of N-desmethylclozapine to said subject, whereby a desired clinical effect is produced. In one embodiment, the subject is human. In some embodiments, the therapeutically effective amount of N-desmethylclozapine is administered as a single dose. In other embodiments, the therapeutically effective amount of N-desmethylclozapine is administered as a plurality of doses. In one embodiment, the dementia manifests as a cognitive impairment. In another embodiment, the dementia manifests as a behavioral disturbance. In one embodiment, the method further comprises administering to the subject an additional therapeutic agent. In one embodiment, the subject is contacted with the additional therapeutic agent subsequent to the contacting with N-desmethylclozapine. In another embodiment, the subject is contacted with the additional therapeutic agent prior to the contacting with N-desmethylclozapine. In still another embodiment, the subject is contacted with the additional therapeutic agent substantially simultaneously with N-desmethylclozapine. In some embodiments, the additional therapeutic agent is selected from the group consisting of selective serotonin reuptake inhibitors, norepinephrine reuptake inhibitors, dopamine agonists, antipsychotic agents, and inverse serotonin 2A agonists.

Also disclosed herein is a method of treating neuropathic pain comprising: identifying a subject suffering from one or more symptoms of neuropathic pain; and contacting said subject with a therapeutically effective amount of N-desmethylclozapine, whereby the symptoms of neuropathic pain are reduced. In one embodiment, the subject is human. In some embodiments, the therapeutically effective amount of N-desmethylclozapine is administered as a single dose. In other embodiments, the therapeutically effective amount of N-desmethylclozapine is administered as a plurality of doses. In one embodiment, the method further comprises contacting the subject with an additional therapeutic agent. In one embodiment, the subject is contacted with the additional therapeutic agent subsequent to the contacting with N-desmethylclozapine. In another embodiment, the subject is contacted with the additional therapeutic agent prior to the contacting with N-desmethylclozapine. In still another embodiment, the subject is contacted with the additional therapeutic agent substantially simultaneously with N-desmethylclozapine. In some embodiments, the additional therapeutic agent is selected from the group consisting of selective serotonin reuptake inhibitors, norepinephrine reuptake inhibitors, dopamine agonists, antipsychotic agents, and inverse serotonin 2A agonists.

Also disclosed herein is a method of treating glaucoma comprising: identifying a subject suffering from one or more symptoms of glaucoma; and contacting said subject with a therapeutically effective amount of N-desmethylclozapine, whereby the symptoms of glaucoma are reduced. In one embodiment, the subject is human. In some embodiments, the therapeutically effective amount of N-desmethylclozapine is administered as a single dose. In other embodiments, the therapeutically effective amount of N-desmethylclozapine is administered as a plurality of doses. In some embodiments, the symptoms of glaucoma are selected from the group consisting of elevated intraocular pressure, optic nerve damage, and decreased field of vision. In one embodiment, the method further comprises contacting the subject with an additional therapeutic agent. In one embodiment, the subject is contacted with the additional therapeutic agent subsequent to the contacting with N-desmethylclozapine. In another embodiment, the subject is contacted with the additional therapeutic agent prior to the contacting with N-desmethylclozapine. In still another embodiment, the subject is contacted with the additional therapeutic agent substantially simultaneously with N-desmethylclozapine. In some embodiments, the additional therapeutic agent is selected from the group consisting of selective serotonin reuptake inhibitors, norepinephrine reuptake inhibitors, dopamine agonists, antipsychotic agents, and inverse serotonin 2A agonists.

Also disclosed herein is a pharmaceutical composition comprising a pharmaceutically effective amount of N-desmethylclozapine and an additional therapeutic agent. In some embodiments, the additional therapeutic agent is selected from the group consisting of selective serotonin reuptake inhibitors, norepinephrine reuptake inhibitors, dopamine agonists, antipsychotic agents, and inverse serotonin 2A agonists. In some embodiments, the antipsychotic agent is selected from the group consisting of a phenothiazine, phenylbutylpiperadine, debenzapine, benzisoxidil, and salt of lithium. In some embodiments, the antipsychotic agent is selected from the group consisting of chlorpromazine (Thorazine®), mesoridazine (Serentil®), prochlorperazine (Compazine®), thioridazine (Mellaril®), haloperidol (Haldol®), pimozide (Orap®), clozapine (Clozaril®), loxapine (Loxitane®), olanzapine (Zyprexa®), quetiapine (Seroquel®), resperidone (Resperidal®), ziprasidone (Geodon®), lithium carbonate, Aripiprazole (Abilify), Clozapine, Clozaril, Compazine, Etrafon, Geodon, Haldol, Inapsine, Loxitane, Mellaril, Moban, Navane, Olanzapine (Zyprexa), Orap, Permitil, Prolixin, Phenergan, Quetiapine (Seroquel), Reglan, Risperdal, Serentil, Seroquel, Stelazine, Taractan, Thorazine, Triavil, Trilafon, Zyprexa, and pharmaceutically acceptable salts thereof. In some embodiments the selective serotonin reuptake inhibitor is selected from the group consisting of fluoxetine, fluvoxamine, sertraline, paroxetine, citalopram, escitalopram, sibutramine, duloxetine, venlafaxine, and pharmaceutically acceptable salts and prodrugs thereof. In some embodiments, the norepinephrine reuptake inhibitor is selected from the group consisting of thionisoxetine and reboxetine. In some embodiments, the dopamine agonist is selected from the group consisting of sumatriptan, almotriptan, naratriptan, frovatriptan, rizatriptan, zomitriptan, cabergoline, amantadine, lisuride, pergolide, ropinirole, pramipexole, and bromocriptine. In one embodiment, the inverse serotonin 2A agonist is N-(1-methylpiperidin-4-yl)-N-(4-flourophenylmethyl)-N'-(4-(2-methylpropyloxy)phenylmethyl)carbamide.

### Brief Description of the Drawings

Figure 1 is a graph showing the results of agonist activity of N-desmethylclozapine at M1 musacrinic acetylcholine receptors in R-SAT Assays.

Figure 2 is a graph showing the results of agonist activity of N-desmethylclozapine at M1 musacrinic acetylcholine receptors in Phosphatidyl Inositol Assay.

Figure 3 shows photographs of MAP kinase activation in rat hippocampus following parenteral administration of N-desmethylclozapine.

### Detailed Description of the Preferred Embodiment

### Definitions

N-desmethylclozapine, 8- chloro -11- (1-piperazinyl) -5H- dibenzo [b,e] [1,4] diazepine, is defined as the compound having the molecular structure depicted in Formula (I).

An "agonist" is defined as a compound that increases the basal activity of a receptor (i.e. signal transduction mediated by the receptor).

An "antagonist" is defined as a compound that competes with an agonist or inverse agonist for binding to a receptor, thereby blocking the action of an agonist or inverse agonist on the receptor. However, an antagonist (also known as a "neutral" antagonist) has no effect on constitutive receptor activity.

A partial agonist is defined as an agonist that displays limited, or less than complete, activity such that it fails to activate a receptor *in vitro,* functioning as an antagonist *in vivo.*

The term "subject" refers to an animal, preferably a mammal, and most preferably a human, who is the object of treatment, observation or experiment.

The term "therapeutically effective amount" is used to indicate an amount of an active compound, or pharmaceutical agent, that elicits the biological or medicinal response indicated. This response may occur in a tissue, system, animal or human that is being sought by a researcher, veterinarian, medical doctor or other clinician, and includes alleviation of the symptoms of the disease being treated.

In certain embodiments, the method disclosed herein includes administering a therapeutically effective amount of NDMC to a subject for the purpose of treating psychosis.

In a further embodiment, the method includes administering a therapeutically effective amount of NDMC to a subject for the purpose of treating depression or mania.

In a still further embodiment, the method includes administering a therapeutically effective amount of NDMC to a subject for the purpose of treating the psychiatric and other behavioral disturbances characteristic of dementia or cognitive impairment of any origin.

In a still further embodiment, the method includes administering a therapeutically effective amount of NDMC to a subject for the purpose of treating neuropathic pain.

The present inventors have profiled a large series of drugs that have utility in treating human disease for functional activity at the five human muscarinic receptor subtypes. With the exception of known muscarinic drugs, only two agents studied (out of more than 500) displayed muscarinic receptor agonist activity. One was the atypical antipsychotic clozapine (8). *In vitro*, this compound has been shown to possess weak partial agonist/antagonist activity at muscarinic M1, M2, and M4 receptors (9, 10), while *in vivo* it is generally considered to display muscarinic receptor antagonist properties. The other was the related compound N-desmethylclozapine.

Administration of clozapine to human subjects results in the formation of two major metabolites N-desmethylclozapine (NDMC) and clozapine-N-oxide (11). However, clozapine-N-oxide is a polar metabolite that is rapidly excreted and likely does not contribute to the biological activity of the parent compound. A correlation exists between the dose of clozapine administered to a subject, and the serum levels of total clozapine moieties, yet the levels of NDMC can vary widely between individual subjects (12). Generally, NDMC constitutes 40-75% of the total serum clozapine concentrations during steady state kinetics in humans (13). Conflicting data exists as to the ability of NDMC to penetrate the blood brain barrier and impart centrally mediated activity (14, 15). These observations demonstrate that NDMC has been routinely administered to human subjects, and is well tolerated. Few data exist as to the molecular properties of NDMC. NDMC has been shown to possess antagonist activity at 5HT_{2C} receptors (16), but no data on its interaction with muscarinic receptors has been reported.

Surprisingly, and unlike the closely related compound clozapine, it has been found that the compound N-desmethylclozapine (NDMC) possesses heretofore unappreciated functional activity as a muscarinic receptor agonist. *Ex vivo* experiments have demonstrated that NDMC crosses the blood brain barrier and acts as an agonist at central muscarinic receptors in rats. These observations have practical applications that support the use of NDMC as an antipsychotic, antimania agent, antidementia agent, and as a therepeutic agent to treat glaucoma or neuropathic pain. Thus, in one aspect, disclosed herein is a method of agonizing the activity of a muscarinic receptor comprising contacting the receptor with an effective amount of NDMC. In another aspect, disclosed herein is a mtehod of treating a subject suffering from a muscarinic receptor related disorder comprising indentifying a subject in need thereof and administering to the subject a therapeutically effective amount of NDMC.

By "muscarinic related disorder," it is meant a disorder whose symptoms are ameliorated by agonizing a muscarinic receptor.

In another aspect, disclosed herein is a method of treating schizophrenia or psychosis of any origin in a subject, comprising identifying a subject in need thereof and administering to the subject a therapeutically effective amount of NDMC. In some embodiments, the method comprises contacting a subject with a pharmacologically active dose of NDMC, for the purpose of controlling the positive (hallucinations and delusion) and negative (apathy, social withdrawal, anhedonia) symptoms of schizophrenia or related psychosis.

In another aspect, disclosed herein is a method of treating affective disorders, including major depression, mania, bipolar disorder, and suicide, in a subject, comprising identifying a subject in need thereof and administering to the subject a therapeutically effective amount of NDMC. In some embodiments, the method comprises contacting a subject with a pharmacologically active dose of NDMC, for the purpose of controlling the symptoms observed during major depression or manic depression.

In another aspect, disclosed herein is a method of treating Alzheimer's Disease and related neurodegenerative disorders in a subject, comprising identifying a subject in need thereof and administering to the subject a therapeutically effective amount of NDMC. In some embodiments, the method comprises contacting a subject with a pharmacologically active dose of NDMC, for the purpose of improving the cognitive deficits, and controlling the associated behavioral abnormalities, observed in degenerative dementias.

In another aspect, disclosed herein is a method of treating neuropathic pain in a subject, comprising identifying a subject in need thereof and administering to the subject a therapeutically effective amount of NDMC. In some embodiments, the method comprises contacting a subject with a pharmacologically active dose of NDMC, for the purpose of controlling the dysthesthetic, hyperalgesic, and other altered nociceptive symptoms observed in neuropathic pain states regardless of their etiology.

In another aspect, disclosed herein is a method of treating glaucoma in a subject, comprising identifying a subject in need thereof and administering to the subject a therapeutically effective amount of NDMC. In some embodiments, the method comprises contacting a subject with a pharmacologically active dose of NDMC, for the purpose of controlling the raised intra-ocular pressure observed in glaucoma, regardless of its etiology.

Surprisingly, NDMC is shown to possess potent agonist activity at the human muscarinic receptors. It is further disclosed herein that NDMC can cross the blood brain barrier, and function *in vivo* as a muscarinic receptor agonist measured via the activation of MAP kinase activity in rat hippocampus. The molecular activities of NDMC, as identified by the present methods, combined with the known clinical efficacy of compounds that possess a similar molecular pharmacological profile, indicate that NDMC can be used to alleviate or treat disorders or conditions associated with human psychosis, affective disease, degenerative dementia, glaucoma, and neuropathic pain.

### Preparation of N-desmethylclozapine (NDMC)

N-desmethylclozapine (NDMC) has the structure of Formula (I).

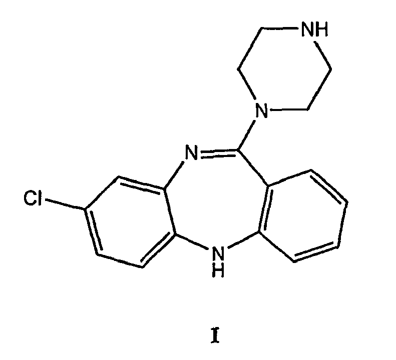

NDMC is prepared as previously described (17). The dibenzo-diazepine-lactam precursor (II) is converted to the thiolactam (III) using phosphorus pentasulfide, followed by alkylation with e.g. dimethyl sulfate to give the imino thioether (IV). Aminolysis of the thioether with an excess of piperazine gives the desired *N*-desmethylclozapine (I). Alternatively, the dibenzo-diazepine-lactam (II) may be converted into the imino-chloride (V) by treatment with a halogenating agent such as phosphorus pentachloride and the product V is converted to N-desmethylclozapine (I) by reaction with piperazine.

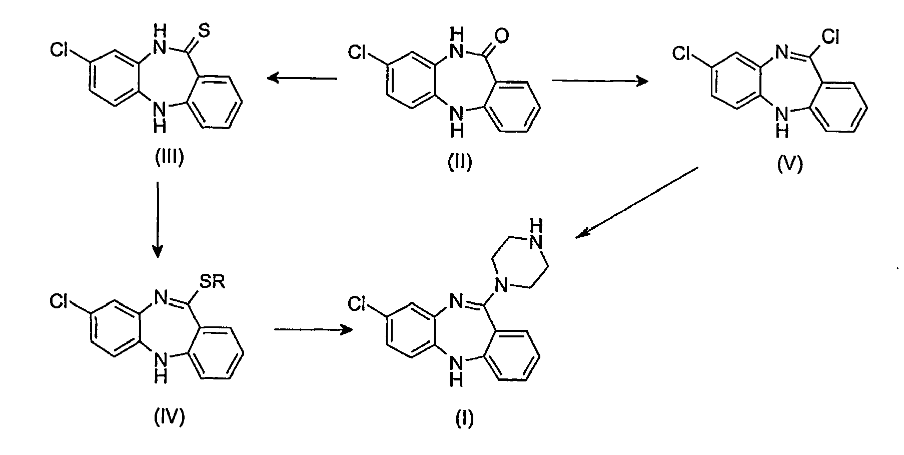

NDMC may be formulated in pharmaceutical compositions comprising NDMC together with a pharmaceutically acceptable dilutant or excipient. Such compositions may be formulated in an appropriate manner and in accordance with accepted practices such as those disclosed in Remington's Pharmaceutical Sciences, Gennaro, Ed., Mack Publishing Co., Easton PA, 1990.

Advantageously, NDMC may be administered in a single daily dose, or the total daily dosage may be administered as a plurality of doses, (*e*.*g*., divided doses two, three or four times daily). Furthermore, compound for the present invention may be administered in intranasal form via topical use of suitable intranasal vehicles, or via transdermal routes, or via topical use of ocular formulations, or using those forms of transdermal skin patches well known to persons skilled in the art.

The dosage regimen of NDMC can be selected in accordance with a variety of factors. These include type, species, age, weight, sex and medical condition of the patient; the severity of the condition to be treated; the route of administration; the renal and hepatic function of the patient; and the particular compound employed. A physician of ordinary skill can readily determine and prescribe the effective amount of the drug required to prevent, counter or arrest the progress of the disease or disorder that is being treated.

The daily dosage of the products may be varied over a wide range from 0.01 to 1000 mg per adult human per day. An effective amount of the drug is ordinarily supplied at a dosage level of about 0.0001 mg/kg to about 25 mg/kg body weight per day. Preferably, the range is from about 0.001 to 10 mg/kg of body weight per day, and especially from about 0.001 mg/kg to 1 mg/kg of body weight per day. The compounds may be administered on a regimen of 1 to 4 times per day.

NDMC may be used alone at appropriate dosages defined by routine testing in order to obtain optimal pharmacological effect, while minimizing any potential toxic or otherwise unwanted effects. In addition, it is believed that NDMC may be used as adjunctive therapy with known drugs to reduce the dosage required of these traditional drugs, and thereby reduce their side effects.

In some embodiments, NDMC is administered in combination with one or more additional therapeutic agents. The additional therapeutic agents can include, but are not limited to, a neuropsychiatric agent. As used herein, a "neuropsychiatric agent" refers to a compound, or a combination of compounds, that affects the neurons in the brain either directly or indirectly, or affects the signal transmitted to the neurons in the brain. Neuropsychiatric agents, therefore, may affect a person's psyche, such as the person's mood, perception, nociception, cognition, alertness, memory, etc. In certain embodiments, the neuropsychiatric agent may be selected from the group consisting of a selective serotonin reuptake inhibitor, norepinephrine reuptake inhibitor, dopamine agonist, antipsychotic agent, and inverse serotonin 2A agonists.

In some embodiments, the antipsychotic agent may be selected from the group consisting of a phenothiazine, phenylbutylpiperadine, debenzapine, benzisoxidil, and salt of lithium. The phenothiazine group of compounds may be selected from the group consisting of chlorpromazine (Thorazine®), mesoridazine (Serentil®), prochlorperazine (Compazine®), and thioridazine (Mellaril®). The phenylbutylpiperadine group of compounds may be selected from the group consisting of haloperidol (Haldol®), and pimozide (Orap®). The debenzapine group of compounds may be selected from the group consisting of clozapine (Clozaril®), loxapine (Loxitane®), olanzapine (Zyprexa®) and quetiapine (Seroquel®). The benzisoxidil group of compounds may be selected from the group consisting of resperidone (Resperidal®) and ziprasidone (Geodon®). The salt of lithium may be lithium carbonate. In some embodiments, the antipsychotic agent may be selected from the group consisting of Aripiprazole (Abilify), Clozapine, Clozaril, Compazine, Etrafon, Geodon, Haldol, Inapsine, Loxitane, Mellaril, Moban, Navane, Olanzapine (Zyprexa), Orap, Permitil, Prolixin, Phenergan, Quetiapine (Seroquel), Reglan, Risperdal, Serentil, Seroquel, Stelazine, Taractan, Thorazine, Triavil, Trilafon, and Zyprexa, or pharmaceutically acceptable salts thereof.

In certain embodiments, the selective serotonin reuptake inhibitor is selected from the group consisting of fluoxetine, fluvoxamine, sertraline, paroxetine, citalopram, escitalopram, sibutramine, duloxetine, and venlafaxine, and pharmaceutically acceptable salts or prodrugs thereof.

In other embodiments, the norepinephrine reuptake inhibitor is selected from the group consisting of thionisoxetine and reboxetine.

In further embodiments, the dopamine agonist is selected from the group consisting of sumatriptan, almotriptan, naratriptan, frovatriptan, rizatriptan, zomitriptan, cabergoline, amantadine, lisuride, pergolide, ropinirole, pramipexole, and bromocriptine.

In another embodiment, the inverse serotonin 2A agonist is N-(1-methylpiperidin-4-yl)-N-(4-flourophenylmethyl)-N'-(4-(2-methylpropyloxy)phenylmethyl)carbamide.

In another aspect, the present disclosure is directed to a method of treating neuropsychiatric disorder in a patient comprising identifying a patient in need thereof and administering to said patient a therapeutically effective amount of a pharmaceutical composition comprising a compound of Formula (I) and a neuropsychiatric agent. In yet another aspect, the present disclosure is directed to a method of treating neuropsychiatric disorder in a patient comprising identifying a patient in need thereof and administering to said patient a therapeutically effective amount of a compound of Formula (I) and a therapeutically effective amount of a neuropsychiatric agent.

In some embodiments, NDMC and additional therapeutic agent(s) are administered nearly simultaneously. These embodiments include those in which the compounds are in the same administrable composition, i.e., a single tablet, pill, or capsule, or a single solution for intravenous injection, or a single drinkable solution, or a single dragee formulation or patch, contains the compounds. The embodiments also include those in which each compound is in a separate administrable composition, but the patient is directed to take the separate compositions nearly simultaneously, i.e., one pill is taken right after the other or that one injection of one compound is made right after the injection of another compound, etc.

In other embodiments, one of NDMC and an additional therapeutic compound is administered first and then the other one of NDMC and the additional therapeutic compound is administered second. In these embodiments, the patient may be administered a composition comprising one of the compounds and then at some time, a few minutes or a few hours later, be administered another composition comprising the other one of the compounds. Also included in these embodiments are those in which the patient is administered a composition comprising one of the compounds on a routine or continuous basis while receiving a composition comprising the other compound occasionally.

Defining the functional pharmacological activity of NDMC at a given receptor can be achieved by a variety of methodologies. A currently favored assay is the Receptor Selection and Amplification Technology (R-SAT) assay disclosed in US 5,707,798.

Defining the functional pharmacological activity of NDMC at a given receptor can be achieved by a variety of methodologies. Another currently favored assay is the PI Hydrolysis assay (18).

Defining the ability of NDMC to penetrate the blood brain barrier and elicit a meaningful biological response can be achieved by a variety of methodologies. A currently favored assay is the hippocampal MAP kinase activation assay (19).

The invention may be described further with reference to the following aspects 1-93:
1. Use of N-desmethylclozapine in the preparation of a medicament for ameliorating psychosis.
2. The use of aspect 1, wherein said medicament is suitable for administration to a human.
3. The use of aspect 1, wherein said medicament is administered as a single dose.
4. The use of aspect 1, wherein said medicament is administered as a plurality of doses.
5. The use of aspect 1, wherein said medicament also comprises an additional therapeutic agent.
6. The use of aspect 1, wherein said additional therapeutic agent is selected from the group consisting of selective serotonin reuptake inhibitors, norepinephrine reuptake inhibitors, dopamine agonists, antipsychotic agents, and inverse serotonin 2A agonists.
7. Use of N-desmethylclozapine in the preparation of a medicament for ameliorating affective disorders.
8. The use of aspect 7, wherein said medicament is suitable for administration to a human.
9. The use of aspect 7, wherein the affective disorder is depression.
10. The use of aspect 7, wherein the affective disorder is mania.
11. The use of aspect 7, wherein said medicament is administered as a single dose.
12. The use of aspect 7, wherein said medicament is administered as a plurality of doses.
13. The use of aspect 7, wherein said medicament also comprises an additional therapeutic agent.
14. The use of aspect 7, wherein said additional therapeutic agent is selected from the group consisting of selective serotonin reuptake inhibitors, norepinephrine reuptake inhibitors, dopamine agonists, antipsychotic agents, and inverse serotonin 2A agonists.
15. Use of N-desmethylclozapine in the preparation of a medicament for ameliorating dementia.
16. The use of aspect 15, wherein said medicament is suitable for administration to a human.
17. The use of aspect 15, wherein said medicament is administered as a single dose.
18. The use of aspect 15, wherein said medicament is administered as a plurality of doses.
19. The use of aspect 15, wherein the dementia manifests as a cognitive impairment.
20. The use of aspect 15, wherein the dementia manifests as a behavioral disturbance.
21. The use of aspect 15, wherein said medicament also comprises an additional therapeutic agent.
22. The use of aspect 15, wherein said additional therapeutic agent is selected from the group consisting of selective serotonin reuptake inhibitors, norepinephrine reuptake inhibitors, dopamine agonists, antipsychotic agents, and inverse serotonin 2A agonists.
23. Use of N-desmethylclozapine in the preparation of a medicament for ameliorating neuropathic pain.
24. The use of aspect 23, wherein said medicament is suitable for administration to a human.
25. The use of aspect 23, wherein said medicament is administered as a single dose.
26. The use of aspect 23, wherein said medicament is administered as a plurality of doses.
27. The use of aspect 23, wherein said medicament also comprises an additional therapeutic agent.
28. The use of aspect 23, wherein said additional therapeutic agent is selected from the group consisting of selective serotonin reuptake inhibitors, norepinephrine reuptake inhibitors, dopamine agonists, antipsychotic agents, and inverse serotonin 2A agonists.
29. Use of N-desmethylclozapine in the preparation of a medicament for ameliorating the symptoms of glaucoma.
30. The use of aspect 29, wherein said medicament is suitable for administration to a human.
31. The use of aspect 29, wherein said medicament is administered as a single dose.
32. The use of aspect 29, wherein said medicament is administered as a plurality of doses.
33. The use of aspect 29, wherein the symptoms of glaucoma are selected from the group consisting of elevated intraocular pressure, optic nerve damage, and decreased field of vision.
34. The use of aspect 29, wherein said medicament also comprises an additional therapeutic agent.
35. The use of aspect 29, wherein said additional therapeutic agent is selected from the group consisting of selective serotonin reuptake inhibitors, norepinephrine reuptake inhibitors, dopamine agonists, antipsychotic agents, and inverse serotonin 2A agonists.
36. A method of treating psychosis comprising:
   identifying a subject suffering from one or more symptoms of psychosis;
   contacting said subject with a therapeutically effective amount of N-desmethylclozapine; whereby the one or more symptoms of psychosis are ameliorated.
37. The method of aspect 36, wherein the subject is human.
38. The method of aspect 36, wherein the therapeutically effective amount of Ndesmethylclozapine is administered as a single dose.
39. The method of aspect 36, wherein the therapeutically effective amount of N-desmethylclozapine is administered as a plurality of doses.
40. The method of aspect 36, further comprising contacting said subject with an additional therapeutic agent.
41. The method of aspect 40, wherein said subject is contacted with said additional therapeutic agent subsequent to said contacting with N-desmethylclozapine.
42. The method of aspect 40, wherein said subject is contacted with said additional therapeutic agent prior to said contacting with N-desmethylclozapine.
43. The method of aspect 40, wherein said subject is contacted with said additional therapeutic agent substantially simultaneously with N-desmethylclozapine.
44. The method of aspect 40, wherein said additional therapeutic agent is selected from the group consisting of selective serotonin reuptake inhibitors, norepinephrine reuptake inhibitors, dopamine agonists, antipsychotic agents, and inverse serotonin 2A agonists.
45. A method of treating affective disorders comprising:
   identifying a subject suffering from one or more symptoms of an affective disorder;
   administering a therapeutically effective amount of N-desmethylclozapine to said subject, whereby the one or more symptoms of the affective disorder are ameliorated.
46. The method of aspect 45, wherein the subject is human.
47. The method of aspect 45, wherein the affective disorder is depression.
48. The method of aspect 45, wherein the affective disorder is mania.
49. The method of aspect 45, wherein the therapeutically effective amount of N-desmethylclozapine is administered as a single dose.
50. The method of aspect 45, wherein the therapeutically effective amount of N-desmethylclozapine is administered as a plurality of doses.
51. The method of aspect 45, further comprising administering to said subject an additional therapeutic agent.
52. The method of aspect 51, wherein said subject is contacted with said additional therapeutic agent subsequent to said contacting with N-desmethylclozapine.
53. The method of aspect 51, wherein said subject is contacted with said additional therapeutic agent prior to said contacting with N-desmethylclozapine.
54. The method of aspect 51, wherein said subject is contacted with said additional therapeutic agent substantially simultaneously with N-desmethylclozapine.
55. The method of aspect 51, wherein said additional therapeutic agent is selected from the group consisting of selective serotonin reuptake inhibitors, norepinephrine reuptake inhibitors, dopamine agonists, antipsychotic agents, and inverse serotonin 2A agonists.
56. A method of treating dementia, comprising:
   identifying a subject suffering from one or more symptoms of dementia;
   administering a therapeutically effective amount of N-desmethylclozapine to said subject, whereby a desired clinical effect is produced.
57. The method of aspect 56, wherein the subject is human.
58. The method of aspect 56, wherein the therapeutically effective amount of N-desmethylclozapine is administered as a single dose.
59. The method of aspect 56, wherein the therapeutically effective amount of N-desmethylclozapine is administered as a plurality of doses.
60. The method of aspect 56, wherein the dementia manifests as a cognitive impairment.
61. The method of aspect 56, wherein the dementia manifests as a behavioral disturbance.
62. The method of aspect 56, further comprising administering to said subject an additional therapeutic agent.
63. The method of aspect 62, wherein said subject is contacted with said additional therapeutic agent subsequent to said contacting with N-desmethylclozapine.
64. The method of aspect 62, wherein said subject is contacted with said additional therapeutic agent prior to said contacting with N-desmethylclozapine.
65. The method of aspect 62, wherein said subject is contacted with said additional therapeutic agent substantially simultaneously with N-desmethylclozapine.
66. The method of aspect 62, wherein said additional therapeutic agent is selected from the group consisting of selective serotonin reuptake inhibitors, norepinephrine reuptake inhibitors, dopamine agonists, antipsychotic agents, and inverse serotonin 2A agonists.
67. A method of treating neuropathic pain comprising:
   identifying a subject suffering from one or more symptoms of neuropathic pain;
   contacting said subject with a therapeutically effective amount of N-desmethylclozapine, whereby the symptoms of neuropathic pain are reduced.
68. The method of aspect 67, wherein the subject is human.
69. The method of aspect 67, wherein the therapeutically effective amount of N-desmethylclozapine is administered as a single dose.
70. The method of aspect 67, wherein the therapeutically effective amount of N-desmethylclozapine is administered as a plurality of doses.
71. The method of aspect 67, further comprising contacting said subject with an additional therapeutic agent.
72. The method of aspect 71, wherein said subject is contacted with said additional therapeutic agent subsequent to said contacting with N-desmethylclozapine.
73. The method of aspect 71, wherein said subject is contacted with said additional therapeutic agent prior to said contacting with N-desmethylclozapine.
74. The method of aspect 71, wherein said subject is contacted with said additional therapeutic agent substantially simultaneously with N-desmethylclozapine.
75. The method of aspect 71, wherein said additional therapeutic agent is selected from the group consisting of selective serotonin reuptake inhibitors, norepinephrine reuptake inhibitors, dopamine agonists, antipsychotic agents, and inverse serotonin 2A agonists.
76. A method of treating glaucoma comprising:
   identifying a subject suffering from one or more symptoms of glaucoma;
   contacting said subject with a therapeutically effective amount of N-desmethylclozapine, whereby the symptoms of glaucoma are reduced.
77. The method of aspect 76, wherein the subject is human.
78. The method of aspect 76, wherein the therapeutically effective amount of N-desmethylclozapine is administered as a single dose.
79. The method of aspect 76, wherein the therapeutically effective amount of N-desmethylclozapine is administered as a plurality of doses.
80. The method of aspect 76, wherein the symptoms of glaucoma are selected from the group consisting of elevated intraocular pressure, optic nerve damage, and decreased field of vision.
81. The method of aspect 76, further comprising contacting said subject with an additional therapeutic agent.
82. The method of aspect 81, wherein said subject is contacted with said additional therapeutic agent subsequent to said contacting with N-desmethylclozapine.
83. The method of aspect 81, wherein said subject is contacted with said additional therapeutic agent prior to said contacting with N-desmethylclozapine.
84. The method of aspect 81, wherein said subject is contacted with said additional therapeutic agent substantially simultaneously with N-desmethylclozapine.
85. The method of aspect 81, wherein said additional therapeutic agent is selected from the group consisting of selective serotonin reuptake inhibitors, norepinephrine reuptake inhibitors, dopamine agonists, antipsychotic agents, and inverse serotonin 2A agonists.
86. A pharmaceutical composition comprising a pharmaceutically effective amount of N-desmethylclozapine and an additional therapeutic agent.
87. The composition of aspect 86 wherein said additional therapeutic agent is selected from the group consisting of selective serotonin reuptake inhibitors, norepinephrine reuptake inhibitors, dopamine agonists, antipsychotic agents, and inverse serotonin 2A agonists.
88. The composition of aspect 87 wherein the antipsychotic agent is selected from the group consisting of a phenothiazine, phenylbutylpiperadine, debenzapine, benzisoxidil, and salt of lithium.
89. The composition of aspect 87, wherein the antipsychotic agent is selected from the group consisting of chlorpromazine (Thorazine®), mesoridazine (Serentil®), prochlorperazine (Compazine®), thioridazine (Mellaril®), haloperidol (Haldol®), pimozide (Orap®), clozapine (Clozaril®), loxapine (Loxitane®), olanzapine (Zyprexa®), quetiapine (Seroquel®), resperidone (Resperidal®), ziprasidone (Geodon®), lithium carbonate, Aripiprazole (Abilify), Clozapine, Clozaril, Compazine, Etrafon, Geodon, Haldol, Inapsine, Loxitane, Mellaril, Moban, Navane, Olanzapine (Zyprexa), Orap, Permitil, Prolixin, Phenergan, Quetiapine (Seroquel), Reglan, Risperdal, Serentil, Seroquel, Stelazine, Taractan, Thorazine, Triavil, Trilafon, Zyprexa, and pharmaceutically acceptable salts thereof.
90. The composition of aspect 87, wherein the selective serotonin reuptake inhibitor is selected from the group consisting of fluoxetine, fluvoxamine, sertraline, paroxetine, citalopram, escitalopram, sibutramine, duloxetine, venlafaxine, and pharmaceutically acceptable salts and prodrugs thereof.
91. The composition of aspect 87, wherein the norepinephrine reuptake inhibitor is selected from the group consisting of thionisoxetine and reboxetine.
92. The composition of aspect 87, wherein the dopamine agonist is selected from the group consisting of sumatriptan, almotriptan, naratriptan, frovatriptan, rizatriptan, zomitriptan, cabergoline, amantadine, lisuride, pergolide, ropinirole, pramipexole, and bromocriptine.
93. The composition of aspect 87, wherein the inverse serotonin 2A agonist is N-(1-methylpiperidin-4-yl)-N-(4-flourophenylmethyl)-N'-(4-(2-methylpropyloxy)phenylmethyl)carbamide.

The present invention is further disclosed in the following example, which is not in any way intended to limit the scope of the invention as claimed.

### Example 1

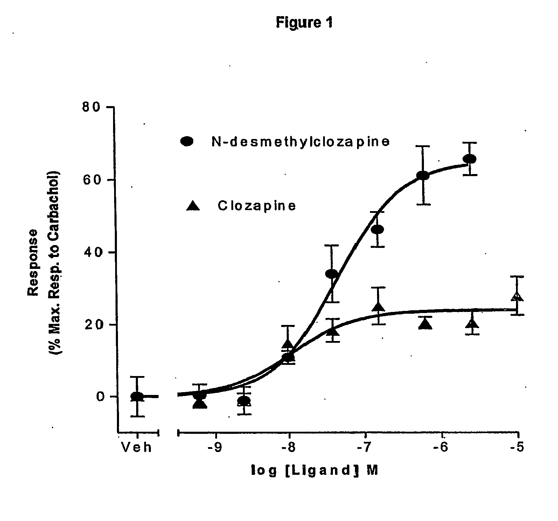

The functional receptor assay, Receptor Selection and Amplification Technology (R-SAT), was used (essentially as disclosed in US 5,707,798) to investigate the functional pharmacological properties of known drugs, including many of their metabolites. These experiments have provided a molecular profile, or fingerprint, for each of these agents. Of all of the agents tested, only one, NDMC, displayed potent M1 acetylcholine receptor agonist activity. Figure 1 shows the concentration response relationship of clozapine (filled triangles) and N-desmethylclozapine (filled circles) to activate human M1 muscarinic receptors. Data was derived from R-SAT assays as previously previously described (20). Data is plotted as the percentage activation relative to the full muscarinic receptor agonist carbachol versus drug concentration. Veh denotes vehicle.

As shwon in Figure 1, clozapine displays high potency (pEC₅₀ of 7.2) yet limited intrinsic efficacy (<25% relative efficacy) at human M1 receptors. Clozapine is thus defined as a weak partial agonist. Partial agonists lack sufficient positive intrinsic activity to stimulate the receptor in a manner similar to full agonists. They thus behave as antagonists *in vivo.* In contrast, NDMC also displays high potency (pEC₅₀ of 7.2) at human M1 receptors, yet it displays significantly greater positive intrinsic activity at M1 receptors (65% relative efficacy to carbachol), behaving as a robust agonist in R-SAT assays. This increased efficacy suggests that NDMC will act as an agonist *in vivo,* a functional profile distinct from that observed for clozapine.

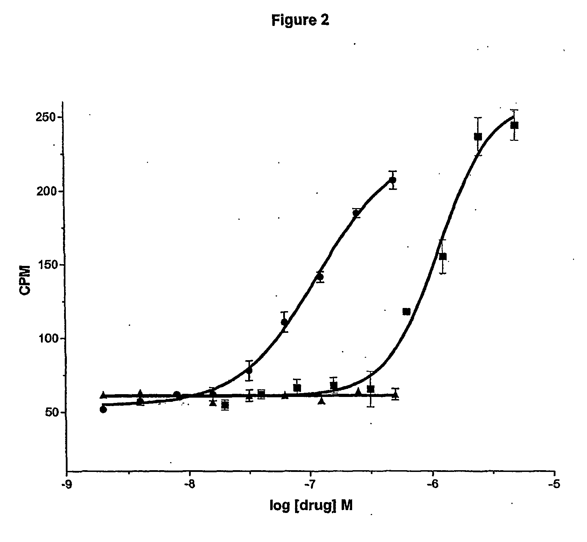

To confirm the observation that NDMC displays increased agonist efficacy at M1 receptors, a PI hydrolysis assay was performed, the results of which are disclosed in Figure 2 and Table 1. The data in Figure 2 is derived from PI assays as described in (18). In Figure 2, the concentration response relationship of carbachol (filled squares), clozapine (filled triangles), and N-desmethylclozapine (filled circles) to activate human M1 muscarinic receptors is shown. Data are plotted as a radioactivity measured in counts per minute versus drug concentration.

**Table 1**

| **Compound** | **M₁** | | |
|---|---|---|---|
| | **%Efficacy** | **pEC50** | **n** |
| Carbachol | 100% | 6.04 ± 0.05 | 5 |
| Clozapine | No Activity | | |
| N-desmethylclozapine | 65 ± 10 | 7.01 ± 0.06 | 5 |

In Table 1, potency is reported as pEC₅₀ values and efficacy is reported as that relative to the full agonist carbachol, both +/- standard deviation. "n" denotes number of experimental determinations. NDMC displays high potency as an M1 agonist in this system (pEC₅₀ = 7.0), with full efficacy (>65% relative efficacy to carbachol). Thus, two distinct functional assays confirm that NDMC possesses previously unappreciated potent and fully efficacious agonist activity at human M1 muscarinic acetylcholine receptors. This significantly greater positive intrinsic activity of NDMC suggests that it behaves as an M1 receptor agonist *in vivo.*

Clozapine and NDMC were tested at the remaining muscarinic receptor subtypes. These data are disclosed in Table 2. The data in Table 2 are derived from R-SAT assays as previously described (20). Potency is reported as pEC₅₀ values and efficacy is reported as that relative to the full agonist carbachol, both +/- standard deviation. N denotes number of experimental determinations.

**Table 2**

| Compound | M₁ | | | M₂ | | | M₃ | | | M₄ | | | M₅ | | |
|---|---|---|---|---|---|---|---|---|---|---|---|---|---|---|---|
| | %Efficacy | pEC50 | N | %Efficacy | pEC50 | N | %Efficacy | pEC50 | N | %Efficacy | pEC50 | N | %Efficacy | pEC50 | N |
| Clozapine | 23±4 | 7.20±0.58 | 4 | 57±4 | 6.41±0.11 | 5 | no response | | 5 | 49±6 | 7.31±0.11 | 6 | no response | | 4 |
| ndesmethylclozapine | 61±5 | 7.22±0.06 | 4 | 78±7 | 6.74±0.22 | 3 | 19±3 | 6.89±0.03 | 3 | 88±3 | 6.63±0.23 | 4 | 66±4 | 7.34±0.15 | 3 |
| Olanzapine | no response | | 3 | no response | | 3 | no response | | 3 | no response | | 4 | no response | | 3 |
| Xanomeline | 121±6 | 7.20 | 17 | 106±9 | 6.30±0.23 | 7 | 66±6 | 6.63±0.21 | 8 | 116±9 | 7.46±0.14 | 5 | 66±12 | 6.59±0.22 | 5 |
| Carbachol | 101±2 | 6.11 | 44 | 101±5 | 6.23±0.09 | 23 | 102±3 | 6.53±0.04 | 27 | 96±3 | 6.53±0.05 | 26 | 105±3 | 6.76±0.12 | 17 |

NDMC displays increased intrinsic activity at all five muscarinic receptor subtypes when compared to clozapine. The profile of NDMC at human muscarinic receptors is most similar to that observed for the investigational agent Xanomeline, with one important distinction, a significantly lower efficacy at human m3 receptors.

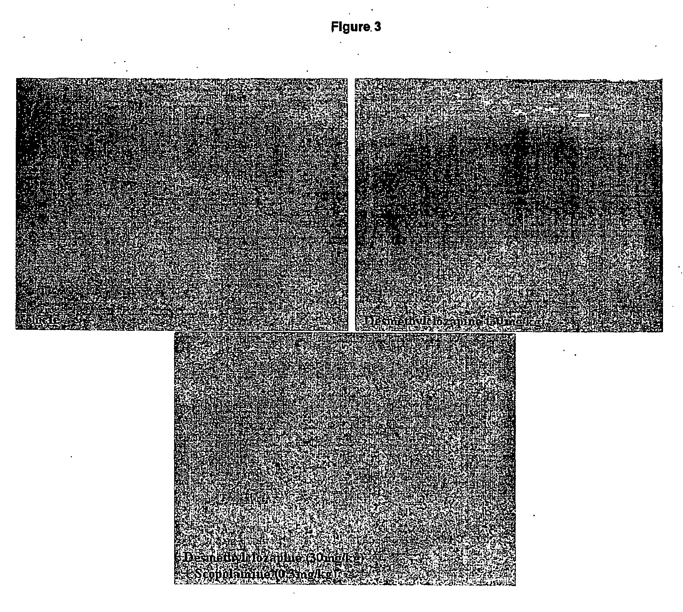

To confirm aspects of this molecular profile *in vivo,* and to assess the ability of NDMC to access the central nervous system, NDMC was administered parenterally to rats, and the M1 receptor mediated activation of hippocampal MAP kinase (MAPK) activity was and this is disclosed in Figure 3. NDMC treatment activates MAPK in CA1 pyramidal neurons. C57BL6 mice were treated s.c with vehicle, N-desmethylclozapine, clozapine, or NDMC and scopolamine (i.p.) at the doses described in Figure 3, and then subjected to labeling via immunohistochemistry. With NDMC treatment, cell bodies and proximal dendrites of CA1 pyramidal neurons showed increased phospho-MAPK immunoreactivity compared to either vehicle or clozapine treatment. Furthermore, scopolamine reduced NDMC induced MAPK activation in the CA1 region indicative of a muscarinic receptor mediated mechanism. Robust activation was observed, at a dose of 30 mg/kg. This confirms that NDMC penetrates the blood brain barrier, and function as a muscarinic receptor agonist *in vivo.*

### Literature Cited

1. Eglen, R., M., Choppin, A., and Watson, N., (2001) Therapeutic opportunities from muscarinic receptor research. Trends Pharmacol. Sci. 22(8): 409-414. [0068]
2. Brown, J., H., and Taylor, P., (1996) Muscarinic receptor agonists and antagonists, in The pharmacological basis of therapeutics. Hardiman, J., G., and Limbird, L., E., editors, Mcgraw-Hill, New York, pp. 141-161.
3. Moroi, S., E., and Lichter, P., R. (1996) Ocular pharmacology, in The pharmacological basis of therapeutics. Hardiman, S., G., and Limbird, L., E., editors, Mcgraw-Hill, New York, pp. 1619-1647.
4. Davis, R E; Doyle, P D; Carroll, R T; Emmerling, M R; Jaen, J. Cholinergic therapies for Alzheimer's disease: Palliative or disease altering? ArzneimittelForschung,.45, 425-431, 1995.
5. Bodick, N., C., Offen, W., W., Levey, A., I., et., al. (1997) Effects of xanomeline, a selective muscarinic receptor agonist, on cognitive function and behavioral symptoms in Alzheimer's disease. Arch. Neurol, 54: 465-473.
6. Shekhar, A., Potter, W., Z., Lienemann, J., et. al. (2001) Efficacy of xanomeline, a selective muscarinic agonist, in treating schizophrenia: a double blind placebo controlled study. ACNP abstracts 135: 173.
7. Rodriquez, M.A., Whipple, B., Ocampo, G., et. al. (2002) Muscarinic agonists in neuropathic and nociceptive pain assays in rats. International Association for the Study of Pain's 10th World Congress, 1160-P76: 388.
8. Baldessarini, R., J., and Frankenburg, F., R. (1991) Clozapine. A novel antipsychotic agent. New. Engl. J. Med., 324(11): 746-754.
9. Olianis, M., C., Maullu, C., and Onali, P., (1999) Mixed agonist-antagonist properties of clozapine at different human cloned muscarinic receptor subtypes expressed in Chinese hamster ovary cells. Neuropsychopharmacology, 20(3): 263-270.
10. Zorn, S., H., Jones, S., B., Ward, K., M., and Liston, D., R. (1994) Clozapine is a potent and selective muscarinic m4 receptor agonist. Eur. J. Pharm. 269: R1-R2.
11. Jann, M., W., Grimsley, S., R., Gray, E., C., and Chang, W. (1993) Pharmacokinetic and pharmacodynamics of clozapine. Clin. Pharmacokinet. 24(2): 161-176.
12. Bondesson, U., and Lindstrom. L., H., (1988) Determination of clozapine and its N-desmethylated metabolite in plasma by use of gas chromatography-mass spectrometry with single ion detection. Psychopharmacology. 95: 472-475.
13. Centorrino, F., Baldessarini, R., J., Kando, J., C., et. al. (1994) Clozapine and metabolites: concentrations in serum and clinical findings during treatment of chronically psychotic patients. J. Clin. Psychopharmacol. 14: 119-125.
14. Baldessarini, R., J., Centorrino, F., Flood, J., G., et. al. (1993) Tissue concentrations of clozapine and its metabolites in the rat. Neuropsychopharmacology. 9(2): 117124.
15. Weigmann, H., Hartter, S., Fischer, V., Dahmen, N., and Hiemke, C. (1999) Distribution of clozapine and desmethylclozapine between blood and brain in rats. European Neuropharmacology 9: 253-256.
16. Kuoppamaki, M., Syvalahti, E., and Hietala, J. (1993) Clozapine and N-desmethylclozapine are potent 5-HT1C receptor antagonists. Eur. J. Pharm. 245: 179-182.
17. Hunziker F. Fisher, E., and Scmutz, J. (1967) 11-amino-5H-dibenzo[b,e]- 1,4-diazepine. Mitteilung uber siebenglienrige Heterocyclen. Helv. China. Acta, 50:1588-1599.
18. Jensen, A., A., Spalding, T., A., Burstein E., S., et. al. (2000) Functional importance of the A1a(116)-Pro(136) region in the calcium-sensing receptor. Constitutive activity and inverse agonism in a family C G-protein-coupled receptor. J Biol Chem. 275(38): 29547-55.
19. Berkeley J., L., Gomeza J., Wess J., Hamilton S., E., Nathanson N., M., and Levey Al. (2001) M1 muscarinic acetylcholine receptors activate extracellular signal-regulated kinase in CA1 pyramidal neurons in mouse hippocampal slices. Mol. Cell Neurosci. 18(5): 512-24.
20. Weiner, D., M., Burstein, E., S., Nash, N., et. al. (2001) 5- hydroxytryptamine 2A receptor inverse agonists as antipsychotics. J Pharmacol Exp Ther., 299(1): 268-76.

## Claims

1. Use of N-desmethylclozapine in the preparation of a medicament for ameliorating psychosis of any origin while agonizing a muscarinic receptor.

2. The use of claim 1, wherein said medicament is suitable for administration to a human.

3. The use of claim 1, wherein said medicament is administered as a single dose.

4. The use of claim 1, wherein said medicament is administered as a plurality of doses.

5. The use of claim 1 for ameliorating positive symptoms of psychosis.

6. The use of claim 5, wherein the positive symptoms include one or more of hallucinations and delusions.

7. The use of claim 1 for ameliorating negative symptoms of psychosis.

8. The use of claim 7, wherein the negative symptoms include one or more of apathy, social withdrawal, and anhedonia.

9. The use of claim 1, wherein the psychosis is associated with schizophrenia.

10. The use of claim 1, wherein the medicament is additionally for treating cognitive impairment of any origin.

11. The use of claim 1, wherein the medicament comprises from 0.01 to 1000 mg of N-desmethylclozapine.

12. The use of claim 1, wherein the muscarinic receptor is a M1 receptor.
